(19) Europäisches Patentamt
European Patent Office
Office européen des brevets

(11) Veröffentlichungsnummer : **0 144 001**
**B1**

(12) # EUROPÄISCHE PATENTSCHRIFT

(45) Veröffentlichungstag der Patentschrift : **06.05.87**

(21) Anmeldenummer : **84113235.0**

(22) Anmeldetag : **03.11.84**

(51) Int. Cl.[4] : **C 07 C129/12**, C 07 C127/26, C 07 C157/14, C 07 D213/64, C 07 D277/68, C 07 D263/58, C 07 D241/44, A 01 N 47/40

(54) **Substituierte Phenoxypropionyl-isoharnstoffderivate, Verfahren zu ihrer Herstellung und ihre Verwendung im Pflanzenschutz.**

(30) Priorität : **11.11.83 DE 3340771**

(43) Veröffentlichungstag der Anmeldung : **12.06.85 Patentblatt 85/24**

(45) Bekanntmachung des Hinweises auf die Patenterteilung : **06.05.87 Patentblatt 87/19**

(84) Benannte Vertragsstaaten : **AT BE CH DE FR GB IT LI NL**

(56) Entgegenhaltungen :
**EP-A- 0 025 010**
**EP-A- 0 061 112**
**ARZNEI.-FORSCH. (DRUG RES.), Band 25, Nr. 10, 1975, Seiten 1477-1482 J.B. BREAM et al.: "Substituted Phenylacetylguanidines: a New Class of Antihypertensive Agents".**

(73) Patentinhaber : **HOECHST AKTIENGESELLSCHAFT**
**Postfach 80 03 20**
**D-6230 Frankfurt am Main 80 (DE)**

(72) Erfinder : **Liebl, Rainer, Dr.**
**An der Weinleite 5b**
**D-8901 Todtenweis (DE)**
Erfinder : **Hörlein, Gerhard, Dr.**
**Assmannshäuser Weg 23**
**D-6000 Frankfurt am Main 71 (DE)**
Erfinder : **Bauer, Klaus, Dr.**
**Kolpingstrasse 7**
**D-6054 Rodgau (DE)**
Erfinder : **Bieringer, Hermann, Dr.**
**Eichenweg 26**
**D-6239 Eppstein/Taunus (DE)**
Erfinder : **Bürstell, Helmut, Dr.**
**Am Hohlacker 65**
**D-6000 Frankfurt am Main 65 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 144 001 B1

Jouve, 18, rue St-Denis, 75001 Paris, France

**Beschreibung**

Gegenstand der vorliegenden Erfindung sind neue Verbindungen der allgemeinen Formel I

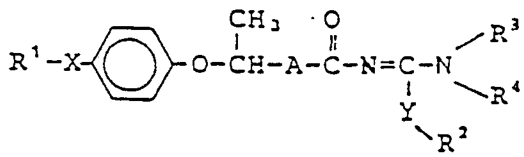

(I)

worin

X O oder $—CH_2—$ ;

Y O, S oder $NR^5$ ;

A eine direkte Bindung, $—CH_2—CH_2—$ oder $—CH=CH—$ ;

$R^1$ die Reste

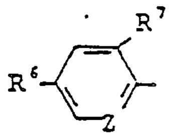 , 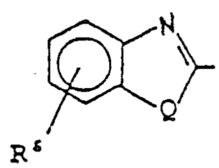 oder 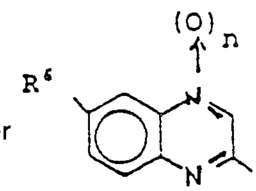

mit

Z = CH, N

Q = O, S

n = 0, 1 ;

$R^2$ $(C_1-C_4)$-Alkyl, wobei für Y = $NR^5$ $R^2$ und $R^5$ zusammen mit dem Stickstoffatom einen 5- oder 6-gliedrigen Ring bilden können, in dem eine —CH-Gruppe durch O oder NH ersetzt sein kann ;

$R^3$ und $R^4$ unabhängig voneinander Wasserstoff oder $(C_1-C_4)$-Alkyl oder gemeinsam die Gruppe

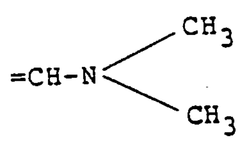 ,

sowie, wenn $R^3$ = Wasserstoff, $R^4$ auch die Gruppen $—CO—R^8$, $COOR^8$ oder $—CO—NHR^8$ ;

$R^5$ Wasserstoff oder $(C_1-C_4)$-Alkyl ;

$R^6$ und $R^7$ unabhängig voneinander H, F, Cl, Br, $CH_3$, $CF_3$, $NO_2$ oder CN ; sowie

$R^8$ $(C_1-C_8)$-Alkyl, $(C_3-C_6)$-Alkenyl, Phenyl, gegebenenfalls ein- bis zweifach substituiert durch Halogen, $CF_3$, $(C_1-C_4)$-Alkyl, bedeuten.

Bevorzugte Verbindungen der allgemeinen Formel I sind dabei Verbindungen in denen X und Y Sauerstoff, A eine direkte Bindung, $R^2$ Methyl oder Ethyl und $R^3$ und $R^4$ jeweils Wasserstoff oder zusammen den Dimethylaminomethylenrest ; $R^6$ $CF_3$, F, Cl oder Br ; $R^7$ H, Cl oder Br ; Z CH oder N ; n Null und Q O oder S bedeuten.

Gegenstand der Erfindung ist auch ein Verfahren zur Herstellung der Verbindungen der allgemeinen Formel I, das dadurch gekennzeichnet ist, daß man Verbindungen der allgemeinen Formel II

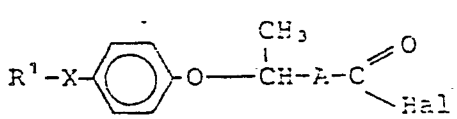

(II)

worin « Hal » Chlor oder Brom bedeutet, mit Verbindungen der allgemeinen Formel III

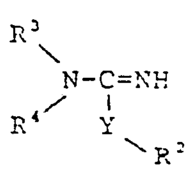

(III)

umsetzt.

Die Verbindungen der allgemeinen Formel II sind bekannt bzw. lassen sich nach bekannte Verfahren, wie sie z. B. in DE-OS 2 623 558, DE-OS 2 914 300, EP-OS 50 019 und BE-PS 889 773 beschrieben werden, herstellen. Die Verbindungen der allgemeinen Formel III sind ebenfalls bekannt ; ihre Herstellung wird z. B. in DE-OS 2 358 904 beschrieben.

Bevorzugt werden die Verbindungen der Formel III in Form ihrer salz- oder schwefelsauren Salze in das erfindungsgemäße Verfahren eingesetzt.

Die Umsetzungen werden in Anwesenheit von anorganischen oder organischen Basen durchgeführt, bevorzugt werden dabei die Alkalihydroxide.

Als Lösungsmittel eignen sich unter den Reaktionsbedingungen inerte Kohlenwasserstoffe oder Halogenkohlenwasserstoffe wie z. B. Toluol, Xylol oder auch Methylenchlorid.

Für den Fall, daß in Methylenchlorid gearbeitet wird, empfiehlt sich der Zusatz von Wasser. Bevorzugt wird dann so verfahren, daß bei Temperaturen von — 10 °C bis + 40 °C z. B. die schwefelsauren Salze der Verbindungen III in einem Gemisch aus Wasser und Methylenchlorid vorgelegt werden und aus getrennten Gefäßen die Verbindungen II sowie die doppelt molare Menge an wäßriger Natronlauge zugetropft werden. Die Aufarbeitung und Isolierung der erfindungsgemäßen Verbindungen erfolgt, je nach Löslichkeit, durch Absaugen bzw. durch Eindampfen der Methylenchloridphase.

Die erfindungsgemäßen Verbindungen der allgemeinen Formel I weisen ein Asymmetriezentrum auf und liegen üblicherweise in racemischer Form vor. Nach dem beschriebenen Herstellverfahren ist es jedoch auch möglich, optisch aktive Ausgangsstoffe der allgemeinen Formel II einzusetzen, deren Herstellung z. B. in der EP-PS 2 800 beschrieben ist. Man gelangt dabei in hohen optischen Reinheiten zu den gewünschten Isomeren. Aufgrund der biologischen Wirksamkeit sind die D-Isomeren bevorzugt. Die allgemeine Formel I umfaßt daher sowohl die Racemate als auch die D-Isomeren der erfindungsgemäßen Verbindungen.

Die Verbindungen der Formel I weisen eine gute herbizide Wirkung und eine gute Selektivität in wichtigen Großkulturen auf. Sie eignen sich zur selektiven Bekämpfung krautiger und grasartiger annueller und perennierender Unkräuter in landwirtschaftlich bedeutenden Kulturen wie z. B. Weizen, Reis, Mais, Zuckerrübe, Soja und Baumwolle im Vor- und Nachauflaufverfahren. Darüber hinaus weisen die erfindungsgemäßen Substanzen gute wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. zur Desikkation, Abszission oder Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen zeigen auch überraschende Wirksamkeit als Herbizid-Antidots (Safener). Sie können somit phytotoxische Effekte, die durch die Anwendung von verschiedenen Herbiziden an Kulturpflanzen entstehen, antagonisieren, d. h. völlig aufheben. Dadurch wird es ermöglicht, bekannte Herbizide selektiv in Kulturen einzusetzen, in denen bisher eine Anwendung nicht möglich war.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können bespielsweise verwendet werden : Alkylarylsulfonsaure Calciumsalze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit fein verteilten, festen Stoffen, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit

Düngemitteln, granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmigen Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Nachstehend seien einige Formulierungsbeispiele aufgeführt :

Beispiel A

Ein Stäubemittel wird erhalten, indem man 10 Gewichtsteile Wirkstoff und 90 Gewichtsteile Talkum als Inertstoff mischt und in einer Schlagmühle zerkleinert.

Beispiel B

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Beispiel C

Ein in Wasser leicht dispergierbares Dispersionskonzentrat wird erhalten, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Beispiel D

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol als Emulgator.

A. Herstellungsbeispiele

Beispiel 1

N-(2-[4-(2,4-Dichlorphenoxy)-phenoxy]-propionyl)-O-methyl-isoharnstoff

6,2 g (0,05 mol) Methylisoharnstoffsulfat werden in 50 ml Wasser und 100 ml Methylenchlorid gelöst und auf 0 °C abgekühlt. Bei 0 °C tropft man gleichzeitig 8 g 50 %ige wäßrige Natronlauge (0,1 mol) und 17,3 g (0,05 mol) 2-[4-(2,4-Dichlorphenoxy)-phenoxy]-propionsäurechlorid, gelöst in 50 ml Toluol, zu. Man rührt 1 h bei 0 °C und 3 h bei 30 °C. Die organische Phase wird abgetrennt, mit 100 ml Wasser gewaschen und über Natriumsulfat getrocknet. Das Lösungsmittel wird unter vermindertem Druck abdestilliert. Man erhält als Rückstand 17,5 g (95 % d. Th.) an N-(2-[4-(2,4-Dichlorphenoxy)-phenoxy]-propionyl)-O-methylisoharnstoff in Form farbloser Kristalle mit Fp 96-97 °C.

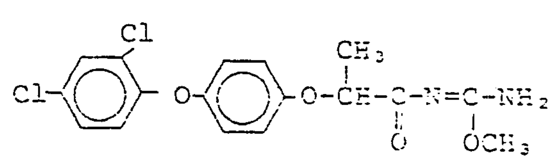

Beispiel 2

N-(2-[4-(4-Chlorbenzyl)-phenoxy]-propionyl)-O-methyl-isoharnstoff

6,2 g (0,05 mol) Methyl-isoharnstoffsulfat werden bei 0 °C in 100 ml Methylenchlorid und 50 ml Wasser gelöst. Man tropft gleichzeitig 15,4 g (0,05 mol) 2-[4-(4-Chlorbenzyl)-phenoxy]-propionsäurechlorid, in 50 ml Toluol gelöst, und 8 g 50 %ige wäßrige Natronlauge (0,1 mol) zu. Man rührt 1 h bei 0 °C und 2 h bei 40 °C und trennt die organische Phase ab. Nach dem Trocknen über Natriumsulfat wird das Lösungsmittel unter vermindertem Druck abdestilliert. Man erhält 13 g (75 % d. Th.) N-(2-[4-(4-Chlorbenzyl)-phenoxy]-propionyl-O-methyl-isoharnstoff in Form eines hellbraunen Öles.

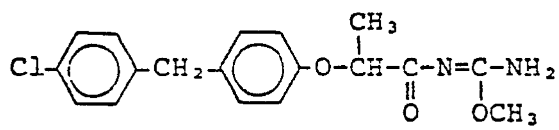

Beispiel 3

N-(2-[4-(4-Trifluormethylphenoxy)-phenoxy]-propionyl)-O-methyl-isoharnstoff

12,3 g (0,1 mol) Methyl-isoharnstoffsulfat werden bei 0 °C in 80 ml Wasser und 150 ml Methylenchlorid gelöst. Unter Eiskühlung tropft man gleichzeitig 34,4 g (0,1 mol) 2-[4-(4-Trifluormethylphenoxy)-phenoxy]-propionsäurechlorid, gelöst in 70 ml Toluol, und 16 g 50 %ige wäßrige Natronlauge (0,2 mol) zu. Man rührt 1 h bei 0 °C und 3 h bei 40 °C, trennt die organische Phase ab, wäscht mit 200 ml Wasser und trocknet über Natriumsulfat. Nach dem Abdestillieren des Lösungsmittels unter vermindertem Druck erhält man 35 g (92 % d. Th.) N-(2-[4-(4-Trifluormethylphenoxy)-phenoxy]-propionyl)-O-methyl-isoharnstoff in Form eines gelben Öles.

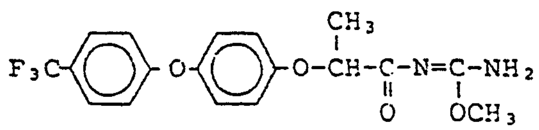

Beispiel 4

N-(2-[4-(6-Chlorbenzthiazol-2-yloxy)-phenoxy)-propionyl)-O-methyl-isoharnstoff

12,3 g (0,1 mol) Methyl-isoharnstoffsulfat werden bei 0 °C in 80 ml Wasser und 150 ml Methylenchlorid gelöst. Bei 0 °C tropft man gleichzeitig unter kräftigem Rühren 36,7 g (0,1 mol) 2-[4-(6-Chlorbenzthiazol-2-yloxy)-phenoxy]-propionsäurechlorid, gelöst in 70 ml Toluol, und 16 g 50 %ige wäßrige Natronlauge (0,2 mol) zu. Man rührt 1 h bei 0 °C und 2 h bei 40 °C. Der ausgefallene Niederschlag wird abgesaugt und mit 50 ml Ether gewaschen. Man erhält 27 g N-(2-[4-(6-Chlorbenzthiazol-2-yloxy)-phenoxy]-propionyl)-O-methyl-isoharnstoff in Form farbloser Kristalle mit Fp. 142 °C. Von der Mutterlauge wird die organische Phase abgetrennt, mit 200 ml Wasser gewaschen und über Natriumsulfat getrocknet. Nach dem Abdestillieren des Lösungsmittels unter vermindertem Druck erhält man einen hellbraunen Feststoff, der mit 100 ml Ether aufgekocht wird. Nach dem Abkühlen saugt man ab und erhält weitere 8,5 g N-(2-[4-(6-Chlorbenzthiazol-2-yloxy)-phenoxy]-propionyl)-O-methyl-isoharnstoff in Form farbloser Kristalle mit Fp 141-142 °C. Gesamtausbeute 35,5 g (88 % d. Th.)

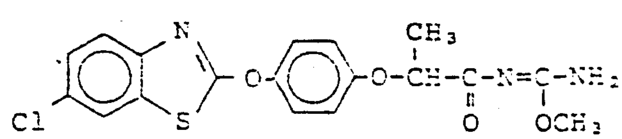

In analoger Weise lassen sich die nachfolgenden, in Tabelle 1 aufgeführten, Verbindungen erhalten.

(Siehe Tabelle 1 Seite 6 f.)

Tabelle 1

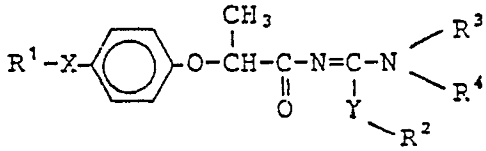

| Bei-spiel Nr. | $R^1$ | X | Y | $R^2$ | $R^3$ | $R^4$ |
|---|---|---|---|---|---|---|
| 5 | 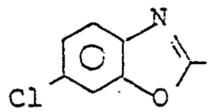 | O | O | $CH_3$ | H | H |
| 6 | " | O | O | $C_2H_5$ | H | H |
| 7 | " | O | S | $CH_3$ | H | H |
| 8 | 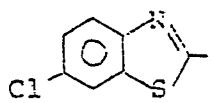 | O | S | $CH_3$ | H | H |
| 9 | " | O | O | $C_2H_5$ | H | H |
| 10 | 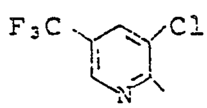 | O | O | $CH_3$ | H | H |
| 11 | 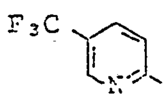 | O | O | $CH_3$ | H | H |
| 12 | 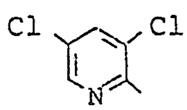 | O | O | $CH_3$ | H | H |
| 13 | 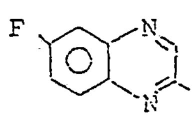 | O | O | $CH_3$ | H | H |
| 14 | 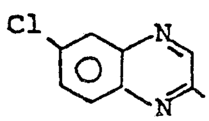 | O | O | $C_2H_5$ | H | H |
| 15 | 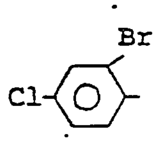 | O | O | $C_2H_5$ | H | H |
| 16 | 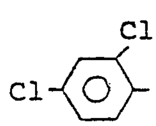 | O | S | $CH_3$ | H | H |

Tabelle 1 (Fortsetzung)

| Beispiel Nr. | $R^1$ | X | Y | $R^2$ | $R^3$ | $R^4$ |
|---|---|---|---|---|---|---|
| 17 | 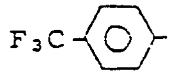 | $-CH_2-$ | -NH | $CH_3$ | H | H |
| 18 | 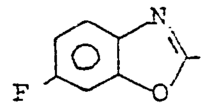 | O | O | $CH_3$ | H | H |
| 19 | 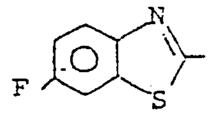 | O | O | $CH_3$ | H | H |

Beispiel 20

N-(2-[4-(2,4-Dichlorphenoxy)-phenoxy]-propionyl)-N'-dimethylaminomethylen-O-methyl-isoharnstoff

19,2 g (0,05 mol) N-(2-[4-(2,4-Dichlorphenoxy)-phenoxy]-propionyl)-O-methyl-isoharnstoff (Beispiel 1) und 9 g (0,06 mol) Dimethylformamid-diethylacetal werden miteinander vermischt. Nach Zugabe von 1/4 ml konz. Schwefelsäure erhitzt man rasch auf 100 °C. Man rührt 3 min bei 100 °C und läßt auf RT abkühlen. Das erhaltene hellbraune Öl wird an der Ölpumpe bei 60-70 °C/0,01 bar von allen flüchtigen Bestandteilen befreit. Man erhält 19,5 g (88 % d. Th.) N-(2-[4-(2,4-Dichlorphenoxy)-phenoxy]-propionyl)-N'-dimethylaminomethylen-O-methyl-isoharnstoff in Form eines hellbraunen, zähen Öles.

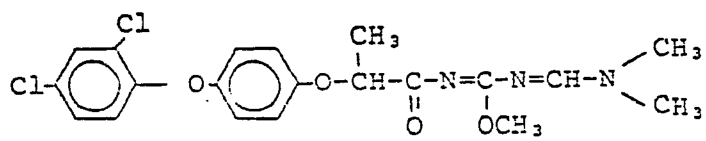

In analoger Weise lassen sich die nachfolgenden, in Tabelle 2 aufgeführten, Verbindungen herstellen.

(Siehe Tabelle 2 Seite 8 f.)

B. Biologische Beispiele

1. Herbizide Wirkung

Die vorliegenden erfindungsgemäßen Verbindungen weisen eine gute herbizide Wirksamkeit gegen ein breites Spektrum wirtschaftlich wichtiger mono- und dikotyler Schadpflanzen auf. Auch schwer bekämpfbare perennierende Arten werden durch die Wirkstoffe gut erfaßt. Dabei ist es gleichgültig, ob die Substanzen in Vorsaat-, Vorauflauf- oder Nachauflaufspritzung ausgebracht werden. Werden die erfindungsgemäßen Verbindungen vor dem Keimen der Unkrautpflanzen im Vorsaat- oder Vorauflaufverfahren auf die Erdoberfläche appliziert, so wird das Auflaufen der Keimlinge nicht vollständig verhindert. Die Unkräuter wachsen bis zum Keimblattstadium heran, stellen jedoch dann ihr Wachstum ein und sterben schließlich nach 3-5 Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann. Obgleich die erfindungsgemäßen Verbindungen auch eine ausgezeichnete herbizide Aktivität besonders gegenüber monokotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur teilweise, oder unwesentlich, oder gar nicht geschädigt. Gegenüber dem Stand der Technik besitzen die vorliegenden erfindungsgemäßen Substanzen deshalb eine wesentlich verbesserte Selektivität bei Kulturpflanzen. Die vorliegenden Verbindungen eignen sich aus diesen Gründen sehr gut zur Bekämpfung von unerwünschtem Pflanzenwuchs in landwirtschaftlichen Nutzpflanzen. Die Schädigung der Unkrautpflanzen bzw. die Kulturpflanzenverträglichkeit wurde in einem Schlüssel von 0-5 bonitiert.

Tabelle 2

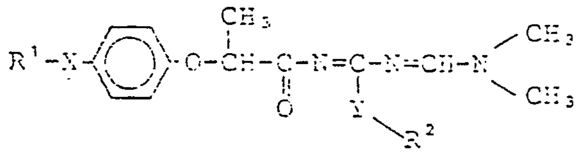

| Beispiel Nr. | $R^1$ | X | Y | $R^2$ |
|---|---|---|---|---|
| 21 | 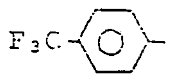 | O | O | $CH_3$ |
| 22 | 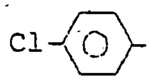 | $-CH_2$ | O | $CH_3$ |
| 23 | 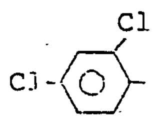 | O | S | $CH_3$ |
| 24 | 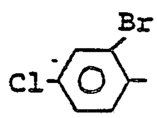 | O | O | $C_2H_5$ |
| 25 | 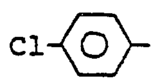 | O | O | $C_2H_5$ |
| 26 | 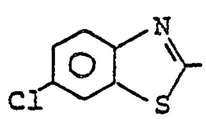 | O | O | $CH_3$ |
| 27 | 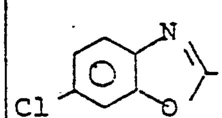 | O | O | $CH_3$ |
| 28 | 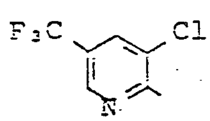 | O | O | $CH_3$ |
| 29 | 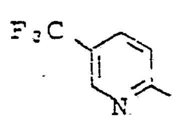 | O | S | $CH_3$ |

Dies bedeutet

0 = ohne Wirkung (Schaden)
1 = 0-20 % Wirkung
2 = 20-40 % Wirkung
3 = 40-60 % Wirkung
4 = 60-80 % Wirkung
5 = 80-100 % Wirkung

a) Wirkung gegen Unkräuter

Samen mono- und dikotyler Unkräuter wurden in Lehmerde in Plastiktöpfen (∅ 9 cm) ausgelegt und mit Erde abgedeckt. Die als benetzbare Pulver bzw. als Emulsionskonzentrate formulierten erfindungsgemäßen Verbindungen wurden in Form wäßriger Suspensionen bzw. Emulsionen auf die Erdoberfläche (Vorauflaufverfahren) bzw. auf die bis zum 4-Blattstadium entwickelten Pflanzen (Nachauflaufverfahren) gespritzt. Die Wasseraufwandmenge pro Topf entsprach dabei umgerechnet 600 l/ha. Nach der Behandlung wurden die Versuchstöpfe im Gewächshaus aufgestellt und die Versuchspflanzen unter guten Wachstumsbedingungen (Temperatur: 23 ± 1 °C; rel. Luftfeuchte 60-80 %) kultiviert. Nach ca. 3 Wochen wurde die Pflanzenschädigung optisch bonitiert. Als Vergleich dienten dabei unbehandelte Kontrollen.

Wie aus den Werten der Tabellen 3 und 4 hervorgeht, zeigen die erfindungsgemäßen Verbindungen im Vorauflauf- bzw. Nachauflaufverfahren sehr gute herbizide Wirksamkeit gegen wirtschaftlich bedeutende mono- und dikotyle Schadpflanzen.

Tabelle 3

Herbizide Wirksamkeit im Vorauflaufverfahren

| Beispiel Nr. | | ECG | LOM | ALM | STM |
|---|---|---|---|---|---|
| 1 | 2,4 | 5 | 5 | - | 4 |
| 1 | 0,6 | 5 | 5 | - | 3 |
| 2 | 2,4 | 5 | 5 | 5 | 5 |
| 2 | 0,6 | 5 | 5 | 5 | 4 |
| 3 | 2,4 | 5 | 5 | 5 | 5 |
| 3 | 0,6 | 5 | 5 | 5 | 4 |

ECG = Echinochloa crus galli
LOM = Lolium multiflorum
ALM = Alopecurus myosuroides
STM = Stellaria media

Selektivität

Die erfindungsgemäßen Verbindungen 1, 2 und 3 sowie 4 werden bei Aufwandmengen von 2,4 kg AS/ha von den Kulturpflanzen Sojabohne, Baumwolle, Zuckerrübe u. a. dikotylen Kulturpflanzen voll toleriert, ohne daß eine Schadwirkung ersichtlich ist. Die Verbindungen 1 und 4 sowie 2 sind darüber hinaus in Getreidearten (Weizen und Reis) voll selektiv.

(Siehe Tabelle 4 Seite 10 f.)

Tabelle 4

Herbizide Wirksamkeit im Nachauflaufverfahren

| Bei-spiel Nr. | | ECG | LOM | ALM |
|---|---|---|---|---|
| 1 | 2,4 | 5 | 5 | - |
| | 0,6 | 5 | 5 | - |
| 2 | 2,4 | 5 | 5 | 5 |
| | 0,6 | 5 | 5 | 5 |
| 3 | 2,4 | 5 | 5 | 4 |
| | 0,6 | 5 | 5 | - |
| 4 | 2,4 | 5 | 5 | 5 |
| | 0,6 | 5 | 5 | 5 |

2. Wirkung als Wachstumsregulator

Zuckerrohrpflanzen werden unter Gewächshausbedingungen bei 25-35 °C und ca. 65 % Luftfeuchtigkeit angezogen. Unterschiedliche Mengen der formulierten Mittel wurden in Wasser suspendiert, das zusätzlich etwa 0,25 Gew.-% eines oberflächenaktiven Mittels (Nonylphenol) enthielt.

Jeweils 0,3 ml der Suspensionen wurden mit Hilfe einer Spritze in den Spindelbereich in Höhe der letzten sichtbaren Blattspreite (« dewlap ») appliziert (10 Pflanzen je Versuchsglied). Das Alter der Pflanzen betrug zum Zeitpunkt der Applikation 6 Monate.

Nach 5 Wochen wurden die Pflanzen geerntet, das Blattwerk entfernt und die 14 oberen Internodien gruppenweise jeweils zusammen im Hinblick auf Saccharosegehalt und Saftreinheit mittels der sog. « press method » (T. Tanimoto, Hawaiin Planters Record, 57, 133 (1964)) analysiert.

Dabei wird der Zuckergehalt polarimetrisch bestimmt und in « Pol Prozent Rohr » ausgedrückt ; diese Zahl entspricht dem Prozentsatz Saccharose in der Lösung unter der Annahme, daß Saccharose die einzige Substanz in der Zuckerlösung ist, die die Ebene des polarisierten Lichts dreht. Die Bestimmung der « Pol % Rohr » ist ein anerkanntes Verfahren zur Bestimmung des Zuckergehaltes von Zuckerrohr.

Die Ergebnisse sind in Tabelle 5 dargestellt.

Tabelle 5

| | Aufwand-menge (mg/ Pflanze) | Saftrein-heit (%) | Saccharose-gehalt (Pol % Rohr) |
|---|---|---|---|
| Kontrolle | | 6,8 | 6,9 |
| Bsp. 3 | 10 | 75 | 11,5 |
| | 5 | 74 | 11,2 |
| Bsp. 4 | 10 | 72 | 10,2 |
| | 5 | 71 | 10,1 |

**Patentansprüche** (für die Vertragsstaaten : BE, CH, DE, FR, GB, IT, LI, NL)

1. Verbindungen der allgemeinen Formel I

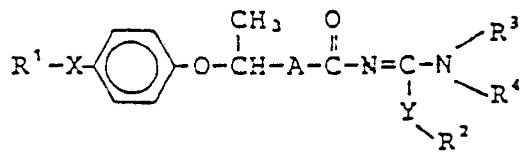

(I)

worin

X O oder $—CH_2—$ ;

Y O, S oder $NR^5$ ;

A eine direkte Bindung, $—CH_2—CH_2—$ oder $—CH{=}CH—$ ;

$R^1$ die Reste

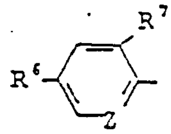

,

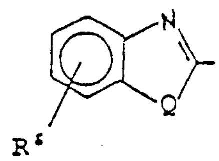

oder

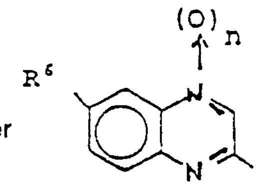

mit

Z = CH, N

Q = O, S

n = 0, 1 ;

$R^2$ ($C_1$-$C_4$)-Alkyl, wobei für Y = $NR^5$ $R^2$ und $R^5$ zusammen mit dem Stickstoffatom einem 5- oder 6-gliedrigen Ring bilden können in dem eine —CH-Gruppe durch O oder NH ersetzt sein kann ;

$R^3$ und $R^4$ unabhängig voneinander Wasserstoff oder ($C_1$-$C_4$)-Alkyl oder gemeinsam die Gruppe

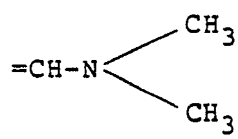

,

sowie, wenn $R^3$ = Wasserstoff, $R^4$ auch die Gruppen $—CO—R^8$, $COOR^8$ oder $—CO—NHR^8$ ;

$R^5$ Wasserstoff oder ($C_1$-$C_4$)-Alkyl ;

$R^6$ und $R^7$ unabhängig voneinander H, F, Cl, Br, $CH_3$, $CF_3$, $NO_2$ oder CN ; sowie

$R^8$ ($C_1$-$C_8$)-Alkyl, ($C_3$-$C_6$)-Alkenyl, Phenyl, gegebenenfalls ein- bis zweifach substituiert durch Halogen, $CF_3$, ($C_1$-$C_4$)-Alkyl, bedeuten.

2. Verbindungen nach Anspruch1, dadurch gekennzeichnet, daß bei Verbindungen in denen X und Y Sauerstoff, A eine direkte Bindung, $R^2$ Methyl oder Ethyl und $R^3$ und $R^4$ jeweils Wasserstoff oder zusammen den Dimethylaminomethylenrest, $R^6$ $CF_3$, F, Cl oder Br ; $R^7$ H, Cl oder Br ; Z CH oder N ; n Null und Q O oder S bedeuten.

3. Verfahren zur Herstellung von Verbindungen der Formel I, dadurch gekennzeichnet, daß man Verbindungen der allgemeinen Formel

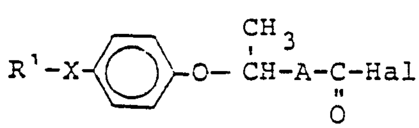

(II)

in denen X, $R^1$, A die oben angegebene Bedeutung haben und « Hal » Chlor oder Brom bedeutet, mit Verbindungen der allgemeinen Formel

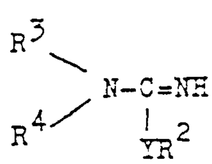

in denen $R^2$, $R^3$, $R^4$, Y die obengenannte Bedeutung haben, umsetzt.

4. Verfahren zur Herstellung der Verbindungen gemäß Anspruch 1-2, dadurch gekennzeichnet, daß man Verbindungen der allgemeinen Formel

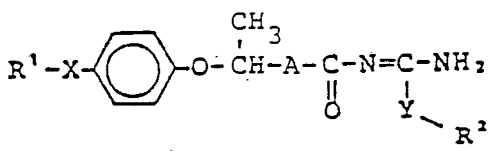

mit Dimethylformamid-dialkylacetalen in Gegenwart saurer Katalysatoren umsetzt.

5. Herbizide Mittel, gekennzeichnet durch einen Gehalt an Verbindungen gemäß Anspruch 1 und 2.

6. Verfahren zur Unkrautbekämpfung in landwirtschaftlichen Kulturen, bzw. zur Beeinflussung des Pflanzenwachstums, dadurch gekennzeichnet, daß man auf die zu behandelnden Flächen bzw. Pflanzen eine wirksame Menge einer Verbindung gemäß Anspruch 1-2 aufbringt.

7. Verfahren zum Schutz vor Kulturpflanzen gegen phytotoxische Nebenwirkungen von Herbiziden, dadurch gekennzeichnet, daß man die Pflanzen, Pflanzenteile oder Böden mit einer Verbindung gemäß Anspruch 1-2 in subtoxischen Konzentrationen, vor, nach oder gleichzeitig mit Herbiziden behandelt.

8. Verwendung von Verbindungen gemäß Anspruch 1-2 zur Bekämpfung von Schadpflanzen, zur Wachstumsregulierung und als toxizitätsmindernde Antidots.

**Patentansprüche** (für den Vertragsstaat AT)

1. Verfahren zur Herstellung von Verbindungen der allgemeinen Formel I

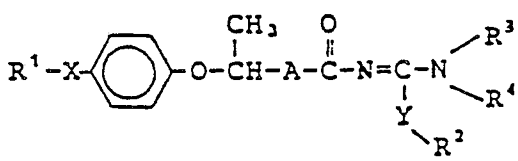

(I)

worin

X O oder —$CH_2$— ;

Y O, S oder $NR^5$ ;

A eine direkte Bindung, —$CH_2$—$CH_2$— oder —CH=CH— ;

$R^1$ die Reste

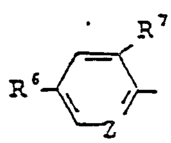 ,

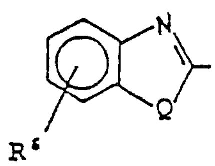

oder

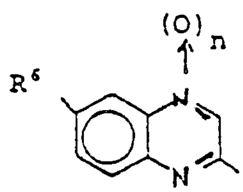

mit

Z = CH, N

Q = O, S

n = 0, 1 ;

$R^2$ ($C_1$-$C_4$)-Alkyl, wobei für Y = $NR^5$ $R^2$ und $R^5$ zusammen mit dem Stickstoffatom einen 5- oder 6-gliedrigen Ring bilden können, in dem eine —CH-Gruppe durch O oder NH ersetzt sein kann ;

$R^3$ und $R^4$ unabhängig voneinander Wasserstoff oder ($C_1$-$C_4$)-Alkyl oder gemeinsam die Gruppe

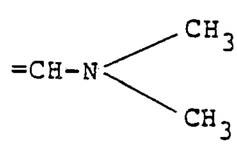 ,

sowie, wenn $R^3$ = Wasserstoff, $R^4$ auch die Gruppen —CO—$R^8$, $COOR^8$ oder —CO—$NHR^8$ ;

$R^5$ Wasserstoff oder ($C_1$-$C_4$)-Alkyl ;

$R^6$ und $R^7$ unabhängig voneinander H, F, Cl, Br, $CH_3$, $CF_3$, $NO_2$ oder CN ; sowie

$R^8$ ($C_1$-$C_8$)-Alkyl, ($C_3$-$C_6$)-Alkenyl, Phenyl, gegebenenfalls ein- bis zweifach substituiert durch Halogen, $CF_3$, ($C_1$-$C_4$)-Alkyl, bedeuten,

bei dem man Verbindungen der allgemeinen Formel II

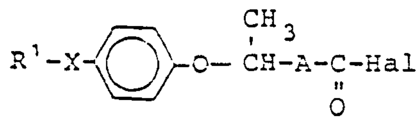

(II)

in denen X, $R^1$, A die oben angegebene Bedeutung haben und « Hal » Chlor oder Brom bedeutet, mit Verbindungen der allgemeinen Formel III

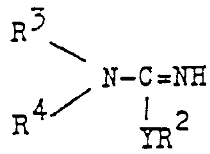

(III)

in denen $R^2$, $R^3$, $R^4$, Y die obengenannte Bedeutung haben, umsetzt.

2. Verfahren zur Herstellung der Verbindungen gemäß Anspruch 1, dadurch gekennzeichnet, daß man Verbindungen der allgemeinen Formel

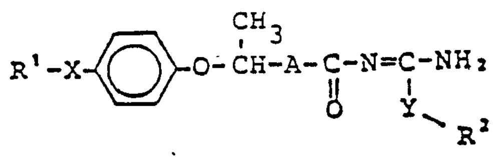

mit Dimethylformamid-dialkylacetalen in Gegenwart saurer Katalysatoren umsetzt.

3. Herbizide Mittel, gekennzeichnet durch einen Gehalt an Verbindungen gemäß der allgemeinen Formel von Anspruch 1.

4. Herbizide Mittel nach Anspruch 3, dadurch gekennzeichnet, daß bei Verbindungen in denen X und Y Sauerstoff, A eine direkte Bindung, $R^2$ Methyl oder Ethyl und $R^3$ und $R^4$ jeweils Wasserstoff oder zusammen den Dimethylaminomethylenrest, $R^6$ $CF_3$, F, Cl oder Br ; $R^7$ H, Cl oder Br ; Z CH oder N ; n Null und Q O oder S bedeuten.

5. Verfahren zur Unkrautbekämpfung in landwirtschaftlichen Kulturen, bzw. zur Beeinflussung des Pflanzenwachstums, dadurch gekennzeichnet, daß man auf die zu behandelnden Flächen bzw. Pflanzen eine wirksame Menge einer Verbindung der allgemeinen Formel von Anspruch 1 aufbringt.

6. Verfahren zum Schutz vor Kulturpflanzen gegen phytotoxische Nebenwirkungen von Herbiziden, dadurch gekennzeichnet, daß man die Pflanzen, Pflanzenteile oder Böden mit einer Verbindung der allgemeinen Formel von Anspruch 1 in subtoxischen Konzentrationen, vor, nach oder gleichzeitig mit Herbiziden behandelt.

7. Verwendung von Verbindungen der allgemeinen Formel von Anspruch 1 zur Bekämpfung von Schadpflanzen, zur Wachstumsregulierung und als toxizitätsmindernde Antidots.

**Claims** (for the Contracting States : BE, CH, DE, FR, GB, IT, LI, NL)

1. A compound of the formula I

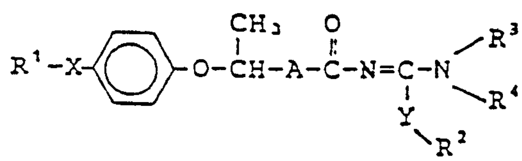

(I)

in which

X denotes O or $—CH_2—$ ;

Y denotes O, S or $NR^5$ ;

A denotes a direct bond, $—CH_2—CH_2—$ or $—CH=CH—$ ;

$R^1$ denotes the radical

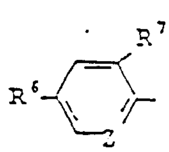

,

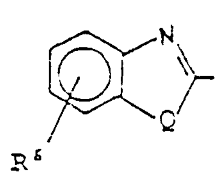

or

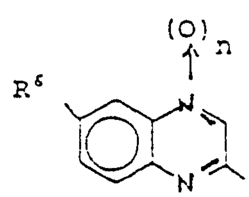

where

Z = CH or N,

Q = O or S and

n = 0 or 1 ;

$R^2$ denotes ($C_1$-$C_4$)-alkyl, where, if Y = $NR^5$, $R^2$ and $R^5$, together with the nitrogen atom, can form a 5-membered or 6-membered ring, in which one —CH-group may be replaced by O or NH ;

$R^3$ and $R^4$ independently of one another denote hydrogen or ($C_1$-$C_4$)-alkyl, or together denote the group

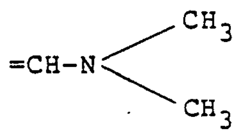

,

and, if $R^3$ = hydrogen, $R^4$ also denotes the group —CO—$R^8$, —$COOR^8$ or —CO—$NHR^8$ ;

$R^5$ denotes hydrogen or ($C_1$-$C_4$)-alkyl ;

$R^6$ and $R^7$ independently of one another denote, H, F, Cl, Br, $CH_3$, $CF_3$, $NO_2$ or CN ; and

$R^8$ denotes ($C_1$-$C_8$)-alkyl, ($C_3$-$C_6$)-alkenyl or phenyl, which can be substituted once or twice by halogen, $CF_3$ or ($C_1$-$C_4$)-alkyl.

2. A compound as claimed in claim 1, in which, if X and Y denote oxygen, A denotes a direct bond, $R^2$ denotes methyl or ethyl and $R^3$ and $R^4$ each denote hydrogen or together denote the dimethylaminomethylene radical, $R^6$ denotes $CF_3$, F, Cl or Br ; $R^7$ denotes H, Cl or Br ; Z denotes CH or N ; n denotes zero ; and Q denotes O or S.

3. A process for the preparation of a compound of the formula I, which comprises reacting a compound of the formula

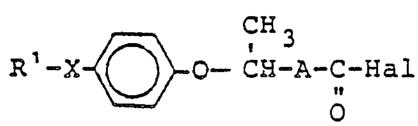

(II)

in which X, $R^1$ and A have the abovementioned meaning, and « Hal » is chloro or bromo, with a compound of the general formula

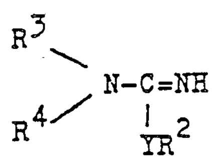

in which $R^2$, $R^3$, $R^4$ and Y have the abovementioned meaning.

4. A process for the preparation of a compound as claimed in either of claims 1 and 2, which comprises reating a compound of the formula

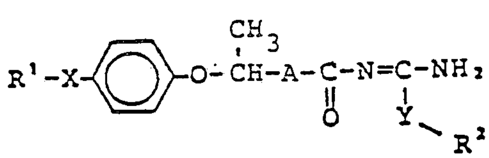

with a dimethylformamide dialkyl acetal in the presence of an acid catalyst.

5. A herbicidal agent containing a compound as claimed in either of claims 1 and 2.

6. A method of combating weeds in agricultural crops and of influencing plant growth, which comprises applying an active amount of a compound of either of claims 1 and 2 to the areas or plants to be treated.

7. A method of protecting crop plants from phytotoxic side effects of herbicides, which comprises treating the plants, part of plants or soil with a compound as claimed in either of claims 1 and 2 in subtoxic concentrations, before, after or at the same time as the treatment with herbicides.

8. The use of a compound as claimed in either of claims 1 and 2 for combating harmful plants, for regulating growth and as a toxicity-reducing antidote.

**Claims** (for the Contracting State AT)

1. A process for the preparation of a compound of the formula I

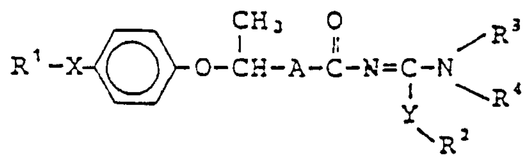

(I)

in which

X denotes O or $—CH_2—$;

Y denotes O, S or $NR^5$;

A denotes a direct bond, $—CH_2—CH_2—$ or $—CH=CH—$;

$R^1$ denotes the radical

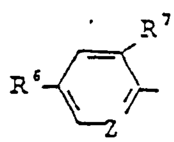

,

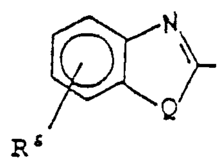

or

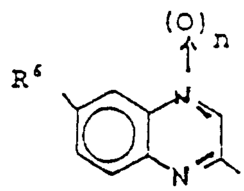

where

Z = CH or N,

Q = O or S and

n = 0 or 1;

$R^2$ denotes $(C_1-C_4)$-alkyl, where, if $Y = NR^5$, $R^2$ and $R^5$, together with the nitrogen atom, can form a 5-membered or 6-membered ring, in which one —CH-group may be replaced by O or NH;

$R^3$ and $R^4$ independently of one another denote hydrogen or $(C_1-C_4)$-alkyl, or together denote the group

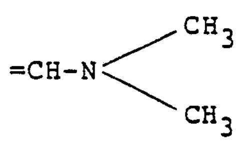

,

and, if $R^3$ = hydrogen, $R^4$ also denotes the group $—CO—R^8$, $—COOR^8$ or $—CO—NHR^8$;

$R^5$ denotes hydrogen or $(C_1-C_4)$-alkyl;

$R^6$ and $R^7$ independently of one another denote, H, F, Cl, Br, $CH_3$, $CF_3$, $NO_2$ or CN; and

$R^8$ denotes $(C_1-C_8)$-alkyl, $(C_3-C_6)$-alkenyl or phenyl, which can be substituted once or twice by halogen, $CF_3$ or $(C_1-C_4)$-alkyl,

which comprises reacting a compound of the formula

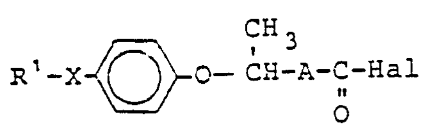

(II)

in which X, $R^1$ and A have the abovementioned meaning, and « Hal » is chloro or bromo, with a compound of the general formula

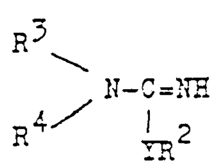

in which $R^2$, $R^3$, $R^4$ and Y have the abovementioned meaning.

2. A process for the preparation of a compound as claimed in claim 1, which comprises reacting a compound of the formula

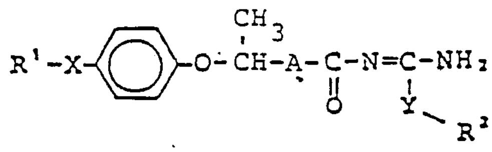

with a dimethylformamide dialkyl acetal in the presence of an acid catalyst.

3. A herbicidal agent containing a compound of the general formula I in which, if X and Y denote oxygen, A denotes a direct bond, $R^2$ denotes methyl or ethyl and $R^3$ and $R^4$ each denote hydrogen or together denote the dimethylaminomethylene radical, $R^6$ denotes $CF_3$, F, Cl or Br ; $R^7$ denotes H, Cl or Br ; Z denotes CH or N ; n denotes zero ; and Q denotes O or S.

4. A herbicidal agent as claimed in claim 3 containing a compound of formula I.

5. A method of combating weeds in agricultural crops and of influencing plant growth, which comprises applying an active amount of a compound of the formula of claim 1 to the areas or plants to be treated.

6. A method of protecting crop plants from phytotoxic side effects of herbicides, which comprises treating the plants, part of plants or soil with a compound of the general formula I of claim 1 in subtoxic concentrations, before, after or at the same time as the treatment with herbicides.

7. The use of a compound of the general formula I of claim 1 for combating harmful plants, for regulating growth and as a toxicity-reducing antidote.

**Revendications** (pour les Etats contractants : BE, CH, DE, FR, GB, IT, LI NL)

1. Les composés de formule générale 1 ci-dessous

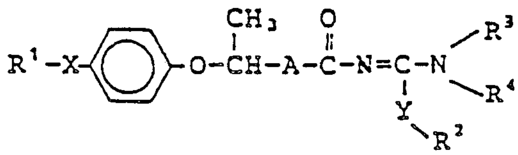

(I)

dans laquelle :

X représente O ou $-CH_2-$ ;

Y représente O, S ou $NR^5$ ;

A représente une liaison directe, $-CH_2-CH_2-$ ou $CH=CH-$ ;

$R^1$ représente un radical

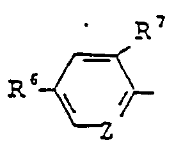

,

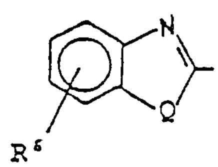

ou

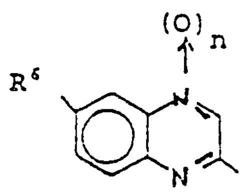

avec

Z = CH, N

Q = O, S

n = 0, 1 ;

$R^2$ un alkyle en $C_1$-$C_4$, et dans le cas où Y est $NR^5$, $R^2$ et $R^5$ peuvent former ensemble et avec l'atome d'azote un cycle pentagonal ou hexagonal dont un groupe —CH— peut être remplacé par O ou NH ; et

$R^3$ et $R^4$ représentent chacun, indépendamment l'un de l'autre, l'hydrogène ou un alkyle en $C_1$-$C_4$ ou bien forment ensemble le groupe

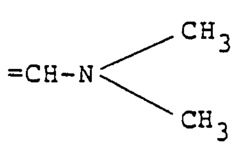

,

et si $R^3$ est l'hydrogène, $R^4$ peut être aussi un groupe $-CO-R^8$, $COOR^8$ ou $-CO-NHR^8$ ;

$R^5$ étant l'hydrogène ou un alkyle en $C_1$-$C_4$ ;

$R^6$ et $R^7$ représentant chacun, indépendamment l'un de l'autre, H, F, Cl, Br, $CH_3$, $CF_3$, $NO_2$ ou CN ; et

$R^8$ étant un alkyle en $C_1$-$C_8$, un alcényle en $C_3$-$C_6$, un phényle portant éventuellement 1 ou 2 atomes d'halogènes ou groupes $CF_3$ ou alkyles en $C_1$ à $C_4$.

2. Composés selon la revendication 1 caractérisés en ce que X et Y sont chacun l'oxygène, A est une liaison directe, $R^2$ un méthyle ou un éthyle et $R^3$ et $R^4$ sont chacun l'hydrogène ou forment ensemble un groupe diméthylaminométhylène, $R^6$ représente $CF_3$, F, Cl ou Br, $R^7$ représente H, Cl ou Br, Z représente CH ou N, n = 0 et Q est un atome d'oxygène ou de soufre.

3. Procédé de préparation des composés de formule I selon la revendication 1 caractérisé en ce que l'on fait réagir des composés de formule II

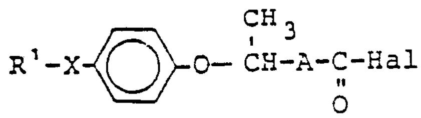

(II)

dans laquelle « Hal » représente le chlore ou le brome, avec des composés de formule III

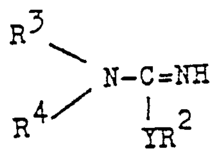

4. Procédé de préparation des composés selon les revendications 1 et 2, caractérisé en ce que l'on fait réagir des composés de formule générale

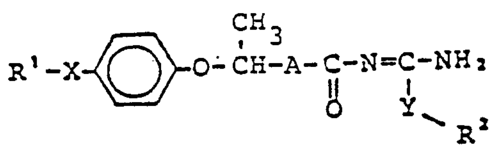

avec des dialkylacétals de diméthylformamide en présence de catalyseurs acides.

5. Produits herbicides caractérisés en ce qu'ils contiennent des composés selon la revendication 1 ou 2.

6. Procédé pour combattre les mauvaises herbes dans des cultures agricoles ou pour agir sur la croissance des plantes, procédé caractérisé en ce que l'on applique sur les surfaces à traiter ou sur les plantes une quantité appropriée d'un composé selon la revendication 1 ou 2.

7. Procédé pour protéger les plantes cultivées contre les effets secondaires phytotoxiques d'herbicides, procédé caractérisé en ce que l'on traite les plantes ou des parties de celles-ci, ou les sols, avec un composé selon la revendication 1 ou 2 à des concentrations sous-toxiques, avant ou après l'application des herbicides, ou en même temps que cette application.

8. L'emploi des composés selon la revendication 1 ou 2 pour lutter contre des plantes nuisibles, comme régulateurs de croissance et comme antidotes diminuant la toxicité d'herbicides.

**Revendications** (pour l'Etat contractant AT)

1. Procédé de préparation de composés de formule générale I :

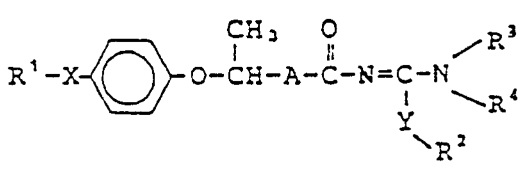

(I)

dans laquelle :

X représente O ou $—CH_2—$ ;

Y représente O, S ou $NR^5$ ;

A représente une liaison directe, $—CH_2—CH_2—$ ou $CH=CH—$ ;

$R^1$ représente un radical

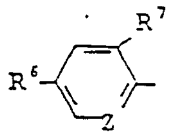 , 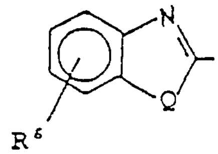 ou 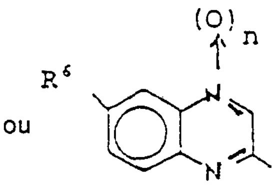

avec

Z = CH, N

Q = O, S

n = 0, 1 ;

$R^2$ un alkyle en $C_1$-$C_4$, et dans le cas où Y est $NR^5$, $R^2$ et $R^5$ peuvent former ensemble et avec l'atome d'azote un cycle pentagonal ou hexagonal dont un groupe —CH— peut être remplacé par O ou NH ; et

$R^3$ et $R^4$ représentent chacun, indépendamment l'un de l'autre, l'hydrogène ou un alkyle en $C_1$-$C_4$ ou bien forment ensemble le groupe

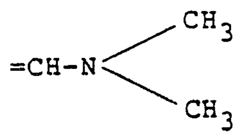 ,

et si $R^3$ est l'hydrogène, $R^4$ peut être aussi un groupe —CO—$R^8$, $COOR^8$ ou —CO—$NHR^8$ ;

$R^5$ étant l'hydrogène ou un alkyle en $C_1$-$C_4$ :

$R^6$ et $R^7$ représentent chacun, indépendamment l'un de l'autre, H, F, Cl, Br, $CH_3$, $CF_3$, $NO_2$ ou CN ; et

$R^8$ étant un alkyle en $C_1$-$C_8$, un alcényle en $C_3$-$C_6$, un phényle portant éventuellement 1 ou 2 atomes d'halogènes ou groupes $CF_3$ ou alkyles en $C_1$ à $C_4$,

procédé dans lequel on fait réagir des composés de formule II

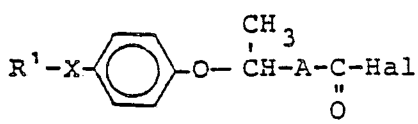 (II)

dans laquelle « Hal » représente le chlore ou le brome, avec des composés de formule III

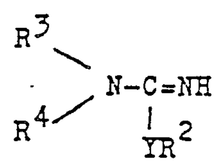

2. Procédé de préparation des composés selon la revendication 1, caractérisé en ce que l'on fait réagir des composés de formule

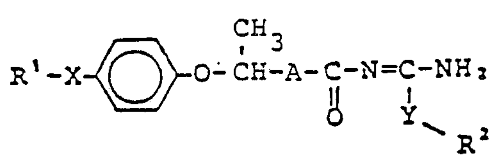

avec des dialkylacétals de diméthylformamide en présence de catalyseurs acides.

3. Produits herbicides caractérisés en ce qu'ils contiennent des composés de formule I selon la revendication 1.

4. Produits herbicides selon la revendication 3 caractérisés en ce qu'ils contiennent des composés dans lesquels X et Y sont chacun l'oxygène, A est une liaison directe, $R^2$ un méthyle ou un éthyle et $R^3$ et $R^4$ sont chacun l'hydrogène ou forment ensemble un groupe diméthylaminométhylène, $R^6$ représente $CF_3$, F, Cl ou Br, $R^7$ représente H, Cl ou Br, Z représente CH ou N, n = 0 et Q est un atome d'oxygène ou de soufre.

5. Procédé pour combattre les mauvaises herbes dans des cultures agricoles ou pour agir sur la croissance des plantes, procédé caractérisé en ce que l'on applique sur les surfaces à traiter ou sur les plantes une quantité appropriée d'un composé de formule selon la revendication 1.

6. Procédé pour protéger des plantes cultivées contre les effets secondaires phytotoxiques d'herbicides, procédé caractérisé en ce que l'on traite les plantes ou des parties de celles-ci, ou les sols,

avec un composé de formule selon la revendication 1 à des concentrations sous-toxiques, avant ou après l'application des herbicides, ou en même temps que cette application.

7. L'emploi des composés de formule I selon la revendication 1 pour combattre des plantes nuisibles, comme régulateurs de la croissance des plantes et comme antidotes diminuant la toxicité d'herbicides.